# EUROPEAN PATENT APPLICATION

(11) **EP 4 437 941 A1**
(43) Date of publication of application: **02.10.2024**
(21) Application number: 24160597.1
(22) Date of filing: 29.02.2024
(51) Int. Cl.: A61B 5/00, A61B 5/024, A61B 5/18, B60W 40/08

(54) **DRIVER ABNORMALITY SIGN DETECTION DEVICE**

(30) Priority: 30.03.2023 JP 2023054813
(71) Applicant: MAZDA MOTOR CORPORATION, Hiroshima 730-8670 (JP)
(72) Inventor: Yoshida, Makoto, Hiroshima, 730-8670 (JP); Nishino, Soma, Hiroshima, 730-8670 (JP)
(74) Representative: Laufhütte, Dieter

(57) **Abstract**

Driver abnormality sign detection method and device are provided that are capable of detecting an abnormality sign of a driver while realizing both of high responsiveness and high reliability.A driver abnormality sign detection method includes a step B of estimating a heartbeat interval BI of a driver based on heartbeats detected by a driver camera, a step C of acquiring time-series data of the heartbeat interval of the driver in a predetermined time period, a step D of standardizing the time-series data of the heartbeat interval, a step E of calculating a probability PA that the standardized heartbeat interval falls within a predetermined range (A region), which has zero as a center, in the time-series data of the standardized heartbeat interval CG-Blz, and a step F of determining that a sign of an abnormality of the driver is detected in a case where the probability PA is greater than a predetermined abnormality sign threshold value TA.

## Description

The present invention relates to driver abnormality sign detection method and device for detecting a sign of an abnormality of a driver who

In related art, a technique has been known which detects a sign of an abnormality of a driver based on a change in driving behavior of the driver. However, in order to detect an abnormality sign of the driver from a change in the driving behavior, the change in the driving behavior has to be monitored for a time period of at least about several minutes, and a case cannot be handled where a driving ability of the driver is lost in a short time (such as epilepsy, a heart attack, or a cerebral stroke, for example).

Accordingly, a technique has been discussed in which by detecting a change in biological functions which lowers the driving ability of the driver, the abnormality sign of the driver is assessed earlier than appearance of the change in the driving behavior. For example, the automatic nervous system configured with the sympathetic nervous system and the parasympathetic nervous system adjusts involuntary biological functions regardless of consciousness of a human, and when an abnormality occurs to the automatic nervous system due to some disease, its influence appears in biological functions, which are controlled by the automatic nervous system, such as circulation, sweating, and pupils, for example. Consequently, it can be considered that by measuring changes in biological information representing states of those biological functions such as a heart rate, a sweating amount, and a pupil diameter, for example, an abnormality of the automatic nervous system can be detected, and the changes can be used for estimation of the abnormality sign of the driver.

In particular, as for the heart rate, from a viewpoint that the heart rate, among pieces of the biological information, is measurable in a contactless manner and an in-vehicle camera mounted on a vehicle can be used as a sensor for the heart rate, detection of an abnormality of the automatic nervous system based on the heart rate of the driver has been discussed.

When use of the heart rate is not limited to the driver, a technique for detecting an abnormality of a living body by using heart rate data has been discussed in related art. For example, a technique has been suggested which detects a symptom of an epileptic seizure by using a heart rate variability index (such as SDNN) obtained by analyzing electrocardiogram data about a time region and a heart rate variability index (such as HF, LF, or LF/HF) obtained by analyzing the electrocardiogram data about a frequency region (for example, see Japanese Patent Laid-Open No. 2015-112423). Further, a technique has been suggested which determines a sudden change in a physical condition by using a biological signal in a VLF band or a ULF band as the heart rate variability index obtained by performing an analysis about the frequency region (for example, see Japanese Patent Laid-Open No. 2020-130264), and a technique has been suggested which detects a state of the heart by performing a nonlinear analysis about a fluctuation in a heart rate variability signal (for example, see Patent Laid-Open No. 2010-184041).

Further, as another heart rate variability index by a non-linear analysis, using a non-Gaussian parameter λ (non-Gaussian index) of a probability density distribution (PDF) has been suggested (for example, see Ken Kiyono, "Non-Gaussian Statistics of Intermittent Fluctuations and Its Application to Heart Rate Variability Analysis" (Journal of the Japan Society for Precision Engineering, Vol. 77, No. 2, 2011, pp. 153 to 157)). In this procedure, a probability density function of heart rate variability of a patient with chronic heart failure and its non-Gaussian index λ are obtained by using a Holter electrocardiogram which is continuously recorded for 24 hours. It has been reported that a significant difference has occurred to the non-Gaussian indices λ obtained from acquired data between survivors and non-survivors in an observation period after data acquisition.

Relevant state of the art is described in Japanese Patent Laid-Open No. 2015-112423, Japanese Patent Laid-Open No. 2020-130264, and Patent Laid-Open No. 2010-184041.

Further state of the art is described in Ken Kiyono, "Non-Gaussian Statistics of Intermittent Fluctuations and Its Application to Heart Rate Variability Analysis" (Journal of the Japan Society for Precision Engineering, Vol. 77, No. 2, 2011, pp. 153 to 157).

However, a method in related art which is disclosed in Japanese Patent Laid-Open No. 2015-112423 has the problem that the method has high responsiveness but is susceptible to noise. That is, a determination about an abnormality is possible based on heart rate data obtained in a comparatively short time, but reliability is low. Further, a method in related art which is disclosed in Patent Laid-Open No. 2020-130264 has the problem that because longer data are necessary for obtaining a biological signal in a VLF band or a ULF band, responsiveness is low. In addition, in a method in related art which is disclosed in Patent Laid-Open No. 2010-184041, in a case where a short time scale is used, results become unstable, and reliability becomes low. In order to make reliability higher, a long time scale has to be used, or plural sets of data in a short time scale have to be acquired through a long period. Either way, there is a problem that it takes time. Further, a method in related art which is disclosed in Japanese Patent Laid-Open No. 2015-112423 has a problem that because data obtained in a long time such as 24 hours have to be acquired, for example, responsiveness is low as well.

Consequently, it is difficult to use the above-described techniques in related art for detection of an abnormality sign of a driver, the detection requesting realization of both of high responsiveness and high reliability.

The present invention has been made for solving such problems, and an object thereof is to provide driver abnormality sign detection method and device that are capable of detecting an abnormality sign of a driver while realizing both of high responsiveness and high reliability.

### [Means for Solving the Problems]

To solve the above-described problems, the present invention provides a driver abnormality sign detection method for detecting a sign of an abnormality of a driver who drives a vehicle, the driver abnormality sign detection method including: a step A of detecting heartbeats of the driver by a heartbeat detection device; a step B of estimating a heartbeat interval of the driver by a processor based on the heartbeats detected by the heartbeat detection device; a step C of acquiring time-series data of the heartbeat interval of the driver in a predetermined time period by the processor; a step D of standardizing the time-series data of the heartbeat interval by the processor; a step E of calculating, by the processor, a probability that the standardized heartbeat interval falls within a predetermined range, which has zero as a center, in the time-series data of the standardized heartbeat interval; and a step F of determining, by the processor, that the sign of the abnormality of the driver is detected in a case where the probability is greater than a predetermined abnormality sign threshold value.

In the present invention configured in such a manner, the processor estimates the heartbeat interval of the driver based on the heartbeats detected by the heartbeat detection device, acquires the time-series data of the heartbeat interval of the driver in the predetermined time period, standardizes the time-series data of the heartbeat interval, and determines that the sign of the abnormality of the driver is detected in a case where the probability that the standardized heartbeat interval falls within the predetermined range, which has zero as the center, in the time-series data of the standardized heartbeat interval is greater than the predetermined abnormality sign threshold value. Thus, the processor acquires the time-series data of the standardized heartbeat interval even from the time-series data of the heartbeat interval in a short time such as about several ten seconds, calculates the probability that the standardized heartbeat interval falls within the predetermined range which has zero as the center, and can thereby detect an abnormality sign. Accordingly, the abnormality sign of the driver can be detected while both of high responsiveness and high reliability are realized.

In the present invention, the predetermined range, which has zero as the center, is preferably included in a central region where a peak of appearance probability distribution of a standardized heartbeat interval acquired from a subject group of subjects having abnormalities in automatic nervous systems protrudes higher than a peak of appearance probability distribution of a standardized heartbeat interval acquired from a subject group of subjects with normal automatic nervous systems.

In the present invention configured in such a manner, a determination about the abnormality sign can be made in a region where clear differences are present between a case where an abnormality is present in the automatic nervous system and a normal case. Accordingly, reliability of abnormality sign detection for the driver can be enhanced.

In the present invention, the abnormality sign threshold value is preferably greater than a value obtained by integrating, by the predetermined range which has zero as the center, the appearance probability distribution of the standardized heartbeat interval acquired from the subject group of subjects with normal automatic nervous systems, and is preferably smaller than a value obtained by integrating, by the predetermined range which has zero as the center, the appearance probability distribution of the standardized heartbeat interval acquired from the subject group of subjects having abnormalities in the automatic nervous systems.

In the present invention configured in such a manner, a boundary between the case where an abnormality is present in the automatic nervous system and the normal case in the predetermined range which has zero as the center is set as the abnormality sign threshold value, and a determination about the abnormality sign can thereby be made. Accordingly, reliability of the abnormality sign detection for the driver can be enhanced.

In the present invention, the step C, the step D, the step E, and the step F are preferably executed at each time when the heartbeat interval is estimated in the step B.

In the present invention configured in such a manner, in a case where the sign of the abnormality of the automatic nervous system of the driver occurs, the sign of the abnormality can quickly be detected. Accordingly, the abnormality sign of the driver can be detected while both of high responsiveness and high reliability are realized.

In another aspect, the present invention provides a driver abnormality sign detection device detecting a sign of an abnormality of a driver who drives a vehicle, the driver abnormality sign detection device including: a heartbeat detection device which detects heartbeats of the driver; a memory which stores a program; and a processor which executes the program, in which the processor is configured to estimate a heartbeat interval of the driver based on the heartbeats detected by the heartbeat detection device, acquire time-series data of the heartbeat interval of the driver in a predetermined time period, standardize the time-series data of the heartbeat interval, calculate a probability that the standardized heartbeat interval falls within a predetermined range, which has zero as a center, in the time-series data of the standardized heartbeat interval, and determine that the sign of the abnormality of the driver is detected in a case where the probability is greater than a predetermined abnormality sign threshold value.

Also in the present invention configured in such a manner, the time-series data of the standardized heartbeat interval can be acquired even from the time-series data of the heartbeat interval in a short time such as about several ten seconds, the probability can be calculated that the standardized heartbeat interval falls within the predetermined range which has zero as the center, and detection of the abnormality sign can thereby be made. Accordingly, the abnormality sign of the driver can be detected while both of high responsiveness and high reliability are realized.

In the present invention, the heartbeat detection device is preferably a driver camera which photographs a body surface of the driver.

In the present invention configured in such a manner, without using a special device for heartbeat detection, the abnormality sign detection for the driver can be performed at a low cost and without a load to the driver.

### [Advantageous Effects of Invention]

Driver abnormality sign detection method and device of the present invention are capable of detecting an abnormality sign of a driver while realizing both of high responsiveness and high reliability.

### Brief Description of Drawings:

FIG. 1 is a block diagram illustrating an outline configuration of a vehicle on which a driver abnormality sign detection device according to an embodiment of the present invention is mounted.
FIG. 2 is a block diagram illustrating a functional configuration of a driver state estimation device according to the embodiment of the present invention.
FIG. 3 is a flowchart of a driver state estimation process according to the embodiment of the present invention.
FIG. 4 is a flowchart of a first parameter learning process according to the embodiment of the present invention.
FIG. 5 is a flowchart of a second parameter learning process according to the embodiment of the present invention.
FIG. 6 is a flowchart of a coarse-graining process about heartbeat intervals according to the embodiment of the present invention.
FIG. 7 is a flowchart of an abnormality sign determination process according to the embodiment of the present invention.
FIG. 8 is a graph representing an appearance probability distribution of a standardized coarse-grained heartbeat interval CG-BIz in each of a subject group of subjects with normal automatic nervous systems and a subject group of subjects having abnormalities in the automatic nervous systems.
FIG. 9 is a flowchart of an abnormality sign determination process according to a second embodiment of the present invention.
FIG. 10 is a time chart representing time changes in heartbeat intervals, coarse-grained heartbeat intervals, and respective probabilities that the coarse-grained heartbeat interval falls into an A region and a B region at a time before an epileptic seizure occurs.
FIG. 11 is a time chart representing time changes in heartbeat intervals, the coarse-grained heartbeat intervals, and the respective probabilities that the coarse-grained heartbeat interval falls into the A region and the B region in a state of sympathetic nervous system failure.
FIG. 12 is a time chart representing time changes in heartbeat intervals, the coarse-grained heartbeat intervals, and the respective probabilities that the coarse-grained heartbeat interval falls into the A region and the B region in a state of parasympathetic nervous system failure.
FIG. 13 is a time chart representing time changes in heartbeat intervals, the coarse-grained heartbeat intervals, and the respective probabilities that the coarse-grained heartbeat interval falls into the A region and the B region of a healthy subject.

### [Mode for Carrying Out the Invention]

Driver abnormality sign detection method and device according to an embodiment of the present invention will hereinafter be described with reference to the attached drawings.

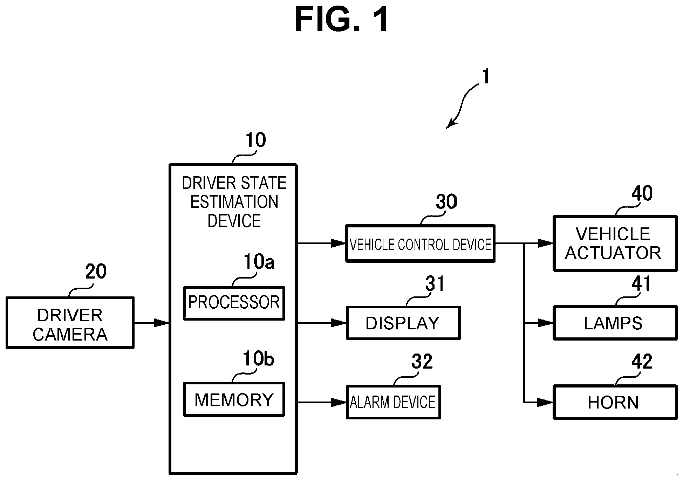

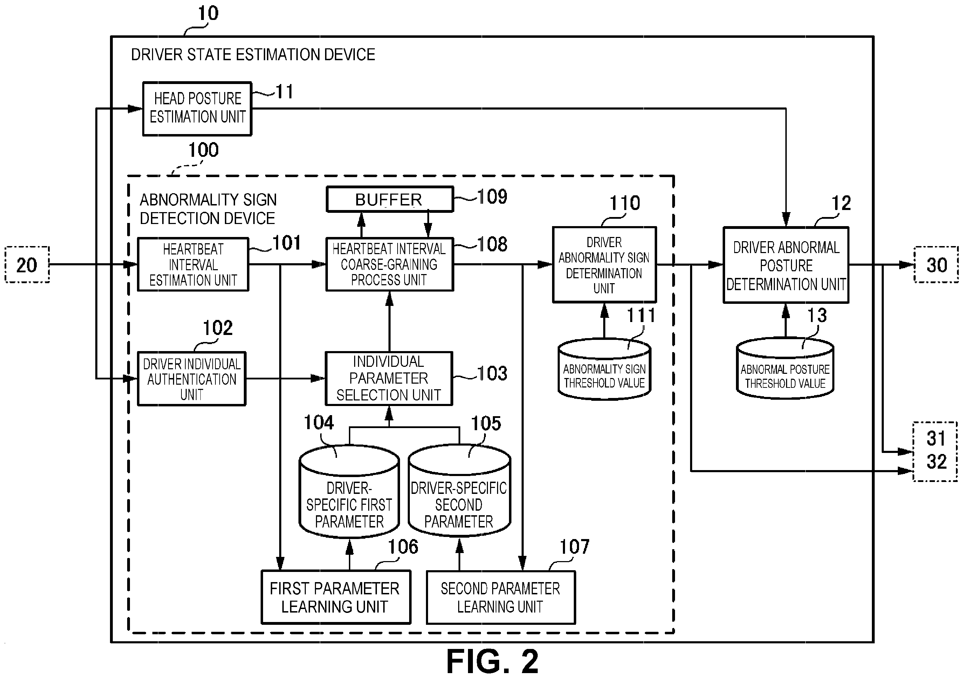

First, a configuration of the driver abnormality sign detection device according to the present embodiment will be described with reference to FIG. 1 and FIG. 2. FIG. 1 is a block diagram illustrating an outline configuration of a vehicle on which the driver abnormality sign detection device is mounted, and FIG. 2 is a block diagram illustrating a functional configuration of a driver state estimation device.

As illustrated in FIG. 1, a vehicle 1 includes a driver state estimation device 10 configured to estimate a state of a driver of the vehicle 1, a driver camera 20 which photographs the driver, a vehicle control device 30 configured to perform control of the vehicle 1 based on information output from the driver state estimation device 10, a display 31 and an alarm device 32 which output information in accordance with control by the driver state estimation device 10, a vehicle actuator 40 which actuates a driving power source, a brake, a steering device, and so forth of the vehicle 1 in accordance with control by the vehicle control device 30, lamps 41 (including headlights, brake lamps, and direction indicators) which act in accordance with control by the vehicle control device 30, and a horn 42. The driver camera 20 photographs a body surface (more specifically, a face surface) of the driver and can thereby detect a pulse wave (heartbeats) in blood vessels close to the body surface from a luminance change in visible light or near infrared rays in an obtained video.

The driver state estimation device 10 mainly has a processor 10a such as one or plural CPUs which execute various processes and one or plural memories 10b (such as a ROM, a RAM, and a hard disk) which store a program to be executed by the processor 10a and various kinds of data necessary for execution of the program.

Next, as illustrated in FIG. 2, the processor 10a of the driver state estimation device 10 functions as a head posture estimation unit 11, a driver abnormal posture determination unit 12, and an abnormality sign detection device 100. The abnormality sign detection device 100 includes a heartbeat interval estimation unit 101, a driver individual authentication unit 102, an individual parameter selection unit 103, a first parameter learning unit 106, a second parameter learning unit 107, a heartbeat interval coarse-graining process unit 108, a buffer 109, and a driver abnormality sign determination unit 110. Further, the memory 10b of the driver state estimation device 10 stores an abnormal posture threshold value 13, a driver-specific first parameter 104, a driver-specific second parameter 105, and an abnormality sign threshold value 111.

Specifically, the head posture estimation unit 11 estimates a posture of a head of the driver based on a video of the driver which is photographed by the driver camera 20. Based on the posture of the head of the driver which is estimated by the head posture estimation unit 11 and on the abnormal posture threshold value 13 stored in the memory 10b, the driver abnormal posture determination unit 12 determines whether or not the posture of the driver is abnormal, controls each unit of the vehicle 1 by the vehicle control device 30 based on a determination result, and causes the display 31 or the alarm device 32 to output an alarm or the like.

The heartbeat interval estimation unit 101 estimates a heartbeat interval of the driver based on the video of the driver which is photographed by the driver camera 20. The driver individual authentication unit 102 specifies the driver by using a face authentication technique based on the video of the driver which is photographed by the driver camera 20.

The individual parameter selection unit 103 acquires, from the memory 10b, a first parameter and a second parameter which correspond to the driver specified by the driver individual authentication unit 102. The heartbeat interval coarse-graining process unit 108 uses the first parameter and the second parameter which are acquired by the individual parameter selection unit 103 and thereby performs a coarse-graining process of the heartbeat interval of the driver which is estimated by the heartbeat interval estimation unit 101.

The first parameter learning unit 106 learns the first parameter specific to the driver based on the heartbeat interval of the driver which is estimated by the heartbeat interval estimation unit 101 and stores the first parameter in the memory 10b. The second parameter learning unit 107 learns the second parameter specific to the driver based on a coarse-grained heartbeat interval calculated by the heartbeat interval coarse-graining process unit 108 and stores the second parameter in the memory 10b.

Based on the coarse-grained heartbeat interval calculated by the heartbeat interval coarse-graining process unit 108 and the abnormality sign threshold value 111 stored in the memory 10b, the driver abnormality sign determination unit 110 determines whether or not a sign of an abnormality of the driver is detected and outputs a determination result to the driver abnormal posture determination unit 12. In a case where the driver abnormality sign determination unit 110 detects the sign of the abnormality of the driver, the driver abnormality sign determination unit 110 causes the display 31 or the alarm device 32 to output an alarm or the like, and the driver abnormal posture determination unit 12 changes the abnormal posture threshold value 13 stored in the memory 10b in a direction in which the posture of the driver is likely to be determined to be abnormal and determines whether or not the posture of the driver is abnormal.

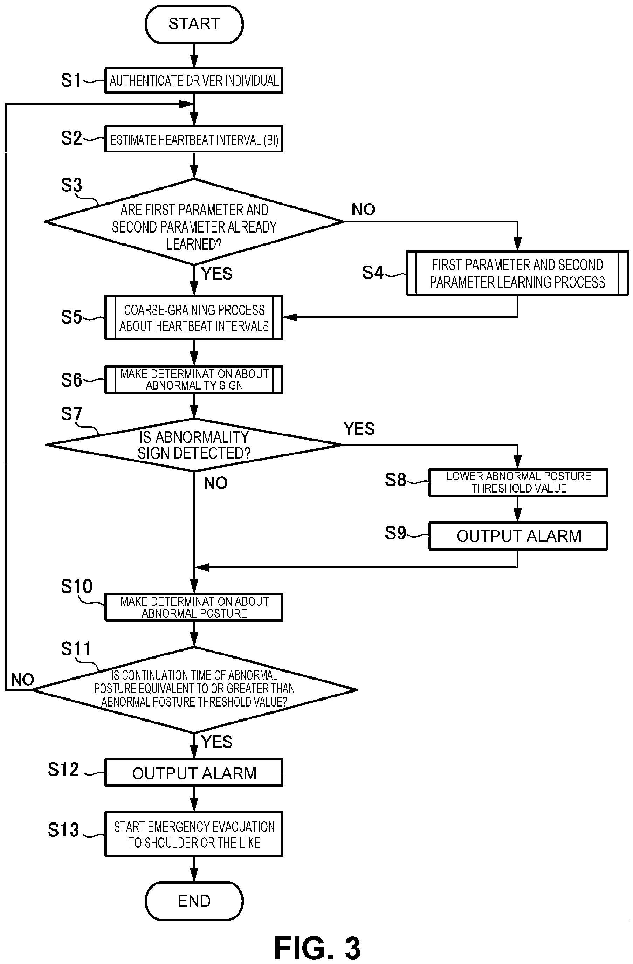

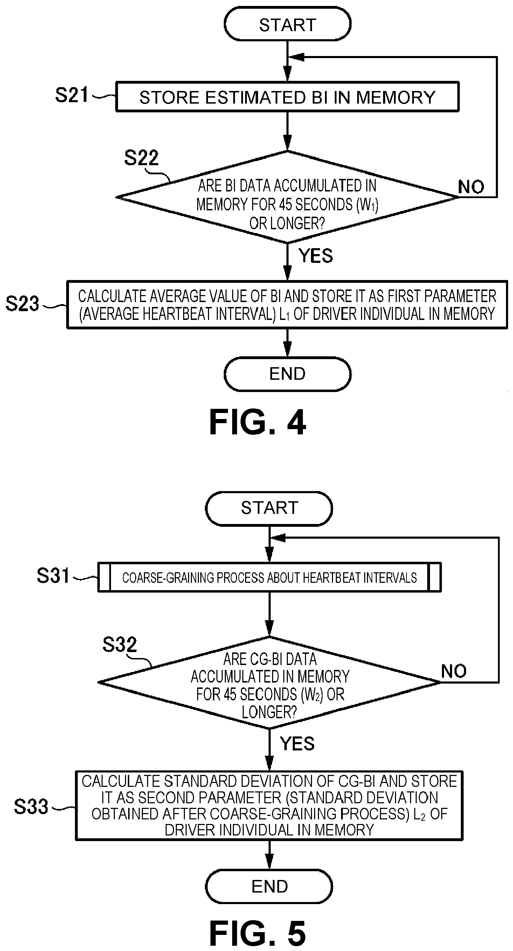

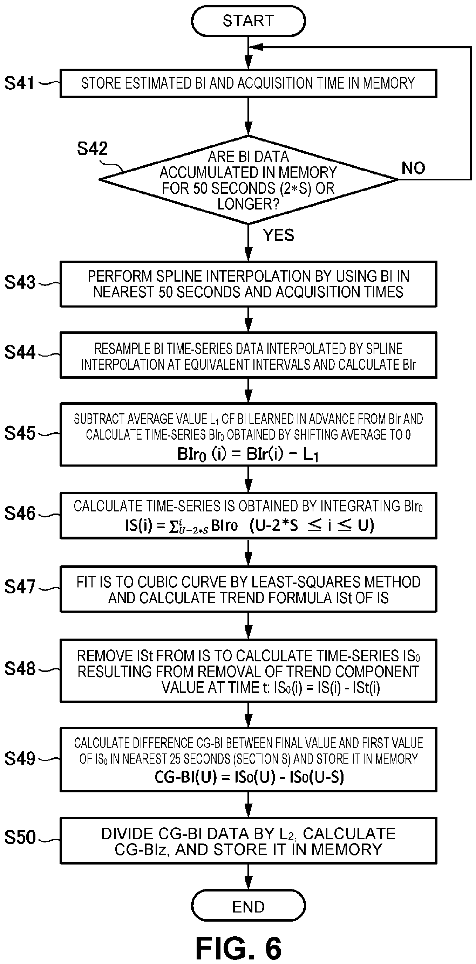

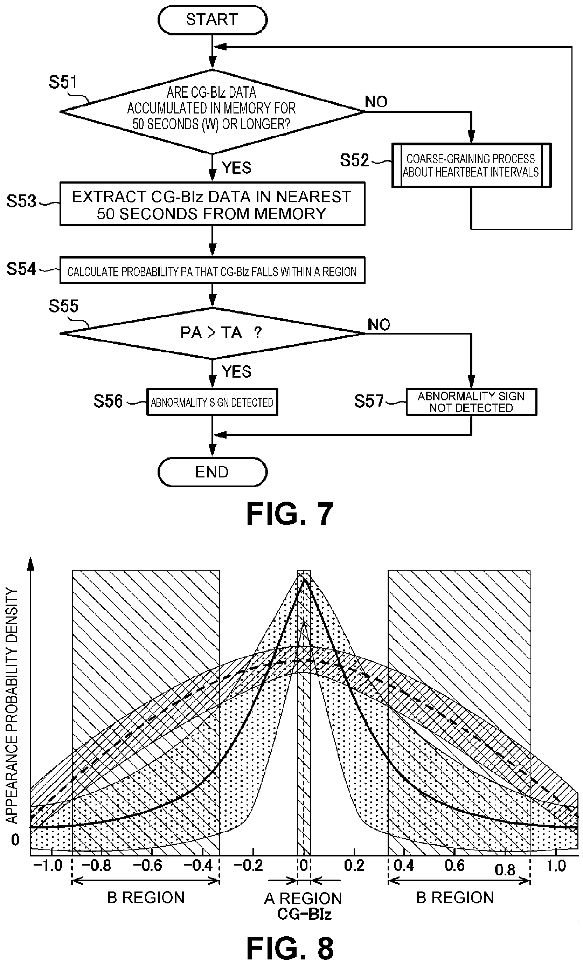

Next, processes to be performed in the above driver state estimation device 10 will be described with reference to FIG. 3 to FIG. 8. FIG. 3 is a flowchart of a driver state estimation process according to the present embodiment. FIG. 4 is a flowchart of a first parameter learning process according to the present embodiment. FIG. 5 is a flowchart of a second parameter learning process according to the present embodiment. FIG. 6 is a flowchart of a coarse-graining process about heartbeat intervals according to the present embodiment. FIG. 7 is a flowchart of an abnormality sign determination process according to the present embodiment. FIG. 8 is a graph representing an appearance probability distribution of a standardized coarse-grained heartbeat interval CG-BIz in each of a subject group of subjects with normal automatic nervous systems and a subject group of subjects having abnormalities in the automatic nervous systems. Note that when descriptions are made about the flowcharts in FIG. 3 to FIG. 7, the processes included in those flowcharts are actually executed by the processor 10a of the driver state estimation device 10, but for easy understanding, the descriptions will be made on the assumption that the processes are executed by function units illustrated by blocks in FIG. 2.

The driver state estimation process illustrated in FIG. 3 is started in a case where an electric power source of the vehicle 1 is turned ON and is finished when the electric power source of the vehicle 1 is turned OFF or after a process of a final step is executed.

First, in step S1, the driver individual authentication unit 102 specifies the driver by using a known face authentication technique based on a video of the driver which is input from the driver camera 20. Face data of each driver which are used for face authentication are in advance acquired by the driver camera 20 and stored in the memory 10b.

Next, in step S2, the heartbeat interval estimation unit 101 estimates the heartbeat interval of the driver based on the video of the driver which is input from the driver camera 20. Specifically, the heartbeat interval estimation unit 101 detects a pulse wave from a video of the body surface of the driver, the video being photographed by the driver camera 20, and estimates a time interval between adjacent peaks in the pulse wave to be a heartbeat interval BI (beat interval).

Next, in step S3, the heartbeat interval coarse-graining process unit 108 determines whether or not the first parameter and the second parameter which are used for a heartbeat interval coarse-graining process are already learned, more specifically, determines whether or not the first parameter and the second parameter of the driver specified in step S1 are stored in the memory 10b.

As a result, in a case where the first parameter and the second parameter are not yet learned (NO in step S3), in step S4, the first parameter learning unit 106 executes the first parameter learning process, and the second parameter learning unit 107 executes the second parameter learning process.

Here, the first parameter learning process will be described with reference to FIG. 4. First, in step S21, the first parameter learning unit 106 stores, in the memory 10b, the heartbeat interval BI estimated in step S2.

Next, in step S22, the first parameter learning unit 106 determines whether or not time-series data of the heartbeat interval BI are accumulated in the memory 10b for a predetermined time period W₁ (for example, 45 seconds) or longer. As a result, in a case where the time-series data of the heartbeat interval BI are not accumulated in the memory 10b for the predetermined time period W₁ or longer (NO in step S22), the process returns to step S21. Subsequently, step S21 and step S22 are repeated until the time-series data of the heartbeat interval BI are accumulated in the memory 10b for the predetermined time period W₁ or longer.

On the other hand, in a case where the time-series data of the heartbeat interval BI are accumulated in the memory 10b for the predetermined time period W₁ or longer (YES in step S22), in step S23, the first parameter learning unit 106 calculates an average value of the heartbeat intervals BI accumulated in the memory 10b and stores, in the memory 10b, the average value as the first parameter of the driver specified in step S1, that is, an average heartbeat interval L₁. Subsequently, the process returns to a main routine of the driver state estimation process.

Next, the second parameter learning process will be described with reference to FIG. 5. First, in step S31, the second parameter learning unit 107 causes the heartbeat interval coarse-graining process unit 108 to execute processes from steps S41 to S49 of a coarse-graining process about the heartbeat intervals BI, which will be described later, and stores a coarse-grained heartbeat interval CG-BI in the memory 10b.

Next, in step S32, the second parameter learning unit 107 determines whether or not time-series data of the coarse-grained heartbeat interval CG-BI are accumulated in the memory 10b for a predetermined time period W₂ (for example, 45 seconds) or longer. As a result, in a case where the time-series data of the coarse-grained heartbeat interval CG-BI are not accumulated in the memory 10b for the predetermined time period W₂ or longer (NO in step S32), the process returns to step S31. Subsequently, step S31 and step S32 are repeated until the time-series data of the coarse-grained heartbeat interval CG-BI are accumulated in the memory 10b for the predetermined time period W₂ or longer.

On the other hand, in a case where the time-series data of the coarse-grained heartbeat interval CG-BI are accumulated in the memory 10b for the predetermined time period W₂ or longer (YES in step S32), in step S33, the second parameter learning unit 107 calculates a standard deviation of the coarse-grained heartbeat intervals CG-BI accumulated in the memory 10b and stores, in the memory 10b, the standard deviation as a second parameter L₂ of the driver specified in step S1. Subsequently, the process returns to the main routine of the driver state estimation process.

Returning to FIG. 3, in a case where the first parameter and the second parameter are already learned in step S3 (YES in step S3) or after the first parameter learning process and the second parameter learning process are executed in step S4, in step S5, the heartbeat interval coarse-graining process unit 108 executes the coarse-graining process about heartbeat intervals.

Here, the coarse-graining process about heartbeat intervals will be described with reference to FIG. 6. First, in step S41, the heartbeat interval coarse-graining process unit 108 stores, in the memory 10b, the heartbeat interval BI estimated in step S2 and an acquisition time of the heartbeat interval BI (for example, a time at which a latter peak is detected, the latter peak being included in adjacent peaks of the pulse wave used for estimation of the heartbeat interval BI) while mutually associating those.

Next, in step S42, the heartbeat interval coarse-graining process unit 108 determines whether or not the time-series data of the heartbeat interval BI are accumulated in the memory 10b for a predetermined time period 2×S or longer. Here, S denotes a coarse-graining time scale, which is used when the heartbeat interval coarse-graining process unit 108 performs coarse graining of the time-series data of the heartbeat interval BI, is in advance decided, and is stored in the memory 10b. In the present embodiment, for example, S = 25 seconds, and in this case, 2×S = 50 seconds.

As a result of the determination in step S42, in a case where the time-series data of the heartbeat interval BI are not accumulated in the memory 10b for the predetermined time period 2×S or longer (NO in step S42), the process returns to step S41. Subsequently, step S41 and step S42 are repeated until the time-series data of the heartbeat interval BI are accumulated in the memory 10b for the predetermined time period 2×S or longer.

On the other hand, in a case where the time-series data of the heartbeat interval BI are accumulated in the memory 10b for the predetermined time period 2×S or longer (YES in step S42), in step S43, the heartbeat interval coarse-graining process unit 108 uses the heartbeat intervals BI in the nearest predetermined time period 2×S (for example, 50 seconds), the heartbeat intervals BI being accumulated in the memory 10b, and the acquisition times of the heartbeat intervals BI and thereby performs spline interpolation for the time-series data of the heartbeat interval BI. Here, when it is assumed that the acquisition time of the newest heartbeat interval BI accumulated in the memory 10b (in other words, the newest detection time of a heartbeat) is set as U, the spline interpolation is performed for the time-series data of the heartbeat interval BI from a time U-2×S to a time U.

Next, in step S44, the heartbeat interval coarse-graining process unit 108 resamples, at equivalent time intervals, the time-series data of the heartbeat interval BI which are interpolated by the spline interpolation in step S43 and thereby acquires time-series data (from the time U-2×S to the time U) of a heartbeat interval BIr which results from resampling.

Next, in step S45, the heartbeat interval coarse-graining process unit 108 subtracts the average heartbeat interval L₁ (the first parameter of the driver), which is learned in the first parameter learning process and stored in the memory 10b, from the resampled heartbeat interval BIr and thereby calculates time-series data (from the time U-2×S to the time U) of a heartbeat interval BIr₀ which is obtained by shifting an average value of the resampled heartbeat intervals BIr to 0.

Next, in step S46, the heartbeat interval coarse-graining process unit 108 calculates time-series data of a value obtained by integration of the time-series data of the heartbeat interval BIr₀, which is obtained by shifting the average value to 0, from the time U-2×S to a time i (U-2×S ≤ i ≤ U), that is, an integrated time-series data IS.

Next, in step S47, the heartbeat interval coarse-graining process unit 108 fits, to a cubic curve by the least-squares method, the integrated time-series data IS (from the time U-2×S to the time U) calculated in step S46 and thereby calculates a trend component ISt of the integrated time-series data IS.

Next, in step S48, the heartbeat interval coarse-graining process unit 108 subtracts the trend component ISt calculated in step S47 from the integrated time-series data IS and thereby calculates integrated time-series data IS₀ (from the time U-2×S to the time U) from which the trend component has been removed.

Next, in step S49, the heartbeat interval coarse-graining process unit 108 calculates a difference CG-BI between the final value (that is, a value IS₀(U) at the time U) and the first value (that is, a value IS₀(U-S) at a time US), in the nearest predetermined time period S, in the integrated time-series data IS₀ calculated in step S48 and stores the difference CG-BI in the memory 10b.

The CG-BI calculated in step S49 is the heartbeat interval CG-BI obtained by performing coarse graining, by a time scale S, for the time-series data of the heartbeat interval BI from the time U-S to the time U. The time scale S is appropriately set (in the present embodiment, S = 25 seconds, for example), the trend component is thereby removed such as a local change in an average heart rate due to an external factor such as a motion or nervousness, and it becomes possible to detect characteristics of variability of the heartbeat interval, the variability reflecting an abnormality of the automatic nervous system of the driver.

Next, in step S50, the heartbeat interval coarse-graining process unit 108 divides the coarse-grained heartbeat interval CG-BI calculated in step S49 by the standard deviation L₂ (the second parameter of the driver) of the coarse-grained heartbeat interval CG-BI which is learned in the second parameter learning process and is stored in the memory 10b, thereby calculates the heartbeat interval CG-BIz obtained by standardization of the coarse-grained heartbeat interval CG-BI, and stores that in the memory 10b. Subsequently, the process returns to the main routine of the driver state estimation process.

Returning to FIG. 3, the heartbeat interval coarse-graining process unit 108 executes the coarse-graining process about heartbeat intervals in step S5, and the driver abnormality sign determination unit 110 thereafter executes the abnormality sign determination process in step S6.

Here, the abnormality sign determination process will be described with reference to FIG. 7. First, in step S51, the driver abnormality sign determination unit 110 determines whether or not time-series data of the standardized coarse-grained heartbeat interval CG-BIz are accumulated in the memory 10b for a predetermined time period W or longer. Here, W denotes a time window for the time-series data of the coarse-grained heartbeat interval CG-BIz, which is referred to when the driver abnormality sign determination unit 110 makes a determination about an abnormality sign of the driver, is in advance decided, and is stored in the memory 10b. In the present embodiment, for example, W = 50 seconds.

As a result, in a case where the time-series data of the standardized coarse-grained heartbeat interval CG-BIz are not accumulated in the memory 10b for the predetermined time period W or longer (NO in step S51), in step S52, the heartbeat interval coarse-graining process unit 108 is caused to execute a coarse-graining process about heartbeat intervals. The coarse-graining process about heartbeat intervals in step S52 is the same as the coarse-graining process about heartbeat intervals in step S5 in the main routine of the driver state estimation process. Subsequently, step S51 and step S52 are repeated until the time-series data of the standardized coarse-grained heartbeat interval CG-BIz are accumulated in the memory 10b for the predetermined time period W or longer.

On the other hand, in a case where the time-series data of the standardized coarse-grained heartbeat interval CG-BIz are accumulated in the memory 10b for the predetermined time period W or longer (YES in step S51), in step S53, the driver abnormality sign determination unit 110 extracts, from the memory 10b, the time-series data of the standardized coarse-grained heartbeat interval CG-BIz in the nearest predetermined time period W.

Next, in step S54, the driver abnormality sign determination unit 110 calculates a probability PA that the CG-BIz falls within a predetermined range (an A region or a central region) which has an average value zero as a center, the CG-BIz being included in time-series data of the standardized coarse-grained heartbeat interval CG-BIz, the time-series data being extracted in step S53.

Here, a setting method of the A region will be described with reference to FIG. 8. FIG. 8 is the graph representing the appearance probability distribution of the standardized coarse-grained heartbeat interval CG-BIz in each of the subject group of subjects with normal automatic nervous systems and the subject group of subjects having abnormalities in the automatic nervous systems. In FIG. 8, the horizontal axis indicates a value of the CG-BIz, and the vertical axis indicates an appearance probability density of each value of the CG-BIz.

In FIG. 8, the broken line represents representative values of the appearance probability distribution of the CG-BIz, which is obtained by executing a coarse-graining process about heartbeat intervals, the coarse-graining process being similar to that described with reference to FIG. 6, for heart rate data of each subject of the subject group of subjects with normal automatic nervous systems (normal group), and a band of rightward-upward oblique lines which extends along the broken line represents a 1σ section of the normal group. Further, in FIG. 8, the solid line represents representative values of the appearance probability distribution of the CG-BIz, which is obtained by executing a coarse-graining process about heartbeat intervals, the coarse-graining process being similar to that described with reference to FIG. 6, for heart rate data of each subject of the subject group of subjects having abnormalities in the automatic nervous systems (abnormal group), and a dotted band which extends along the solid line indicates a 1σ section of the abnormal group.

When the appearance probability distributions of the CG-BIz in the normal group and the abnormal group are compared with each other, as illustrated in FIG. 8, a kurtosis of the appearance probability distribution of the CG-BIz in the abnormal group is higher than a kurtosis of the appearance probability distribution of the CG-BIz in the normal group. That is, a peak of the appearance probability density of the CG-BIz of the abnormal group protrudes higher than that of the appearance probability density of the CG-BIz of the normal group in a region in the vicinity of a central portion, the region including the average value zero of the CG-BIz, and the appearance probability density of the CG-BIz of the abnormal group is lower than the appearance probability density of the CG-BIz of the normal group in peripheral regions on both sides and outsides of the region in the vicinity of the central portion. This can be considered to be because in the normal group, sympathetic nerves and parasympathetic nerves work to mutually keep balance, and the heartbeat interval thereby fluctuates, but in the abnormal group, an abnormality occurring to either one of the sympathetic nerves and the parasympathetic nerves makes their balance unstable, an abnormality occurring to both of the sympathetic nerves and the parasympathetic nerves causes a change in biological functions to disappear, and the fluctuation of the heartbeat interval thereby becomes small. Accordingly, the inventors of the present application have considered that the abnormality sign of the driver due to failure of the automatic nervous system can be detected based on a difference in the appearance probability distribution of the CG-BIz between the normal group and the abnormal group in the region in the vicinity of the central portion, the region including the average value zero of the CG-BIz.

Specifically, in a range in the vicinity of the central portion, the range including the average value 0 of the CG-BIz, a range, in which the 1σ section of the appearance probability distribution of the CG-BIz of the normal group (the band of rightward-upward oblique lines) does not overlap the 1σ section of the appearance probability distribution of the CG-BIz of the abnormal group (the dotted band), (for example, a range in which the CG-BIz is -0.03 or greater to 0.03 or smaller) is set as the A region (central region). Furthermore, in a case where the probability PA that the CG-BIz calculated from the heartbeat interval of the driver falls within the A region is higher than an abnormality sign threshold value TA, the driver abnormality sign determination unit 110 determines that the abnormality sign of the driver is detected. The abnormality sign threshold value TA is set to a value which is higher than a probability that a representative value +1σ of the appearance probability distribution of the CG-BIz of the normal group falls within the A region and which is lower than a probability that a representative value -1σ of the appearance probability distribution of the CG-BIz of the abnormal group falls within the A region.

That is, the abnormality sign threshold value TA is set to a value which is greater than a value obtained by integrating a curve representing the representative value +1σ of the appearance probability distribution of the CG-BIz of the normal group (a curve demarcating an upper side of the band of rightward-upward oblique lines) by the A region (that is, the range in which the CG-BIz is -0.03 or greater to 0.03 or smaller) and which is smaller than a value obtained by integrating a curve representing the representative value - 1σ of the appearance probability distribution of the CG-BIz of the abnormal group (a curve demarcating a lower side of the dotted band) by the A region, and TA = 0.09, for example. A numerical value range of the CG-BIz which defines the A region and the abnormality sign threshold value TA are in advance set based on the heart rate data of the normal group and the abnormal group and are stored in the memory 10b.

Returning to FIG. 7, in step S55, the driver abnormality sign determination unit 110 determines whether or not the probability PA calculated in step S54 is greater than the abnormality sign threshold value TA (that is, a probability TA that the representative value -1σ of the appearance probability distribution of the CG-BIz of the abnormal group falls within the A region) stored in the memory 10b.

As a result, in a case where the PA is greater than the TA (YES in step S55), the driver abnormality sign determination unit 110 determines that the abnormality sign of the driver is detected in step S56. On the other hand, in a case where the PA is the TA or smaller (NO in step S55), the driver abnormality sign determination unit 110 determines that the abnormality sign of the driver is not detected in step S57. After step S56 or step S57, the process returns to the main routine of the driver state estimation process.

Returning to FIG. 3, after the abnormality sign determination process is executed in step S6, in step S7, the driver abnormal posture determination unit 12 determines whether or not the abnormality sign of the driver is detected in the abnormality sign determination process. As a result, in a case where the abnormality sign of the driver is detected (YES in step S7), in step S8, the driver abnormal posture determination unit 12 changes the abnormal posture threshold value stored in the memory 10b in the direction in which the posture of the driver is likely to be determined to be abnormal. For example, in a case where it is determined that the posture of the driver is abnormal when an angle of the head of the driver is a first abnormal posture threshold value or greater and a duration of this state is a second abnormal posture threshold value or greater, the driver abnormal posture determination unit 12 lowers either one or both of the first abnormal posture threshold value and the second abnormal posture threshold value.

Next, in step S9, the driver abnormal posture determination unit 12 causes the display 31 or the alarm device 32 to output an alarm which notifies that the abnormality sign of the driver is detected.

After step S9 or in a case where the abnormality sign of the driver is not detected in step S7 (NO in step S7), in step S10, the driver abnormal posture determination unit 12 compares the posture of the head of the driver, the posture being estimated by the head posture estimation unit 11, with the first abnormal posture threshold value and thereby determines whether or not the posture of the driver is abnormal.

Next, in step S11, in a case where it is determined that the posture of the driver is abnormal in step S10, the driver abnormal posture determination unit 12 determines whether or not a duration of the above abnormal posture is equivalent to or greater than the second abnormal posture threshold value stored in the memory 10b. As a result, in a case where the duration of the abnormal posture is smaller than the second abnormal posture threshold value (NO in step S11), the process returns to step S2. Subsequently, while the duration of the abnormal posture is smaller than the second abnormal posture threshold value, processes in steps S2 to S11 are repeated. That is, at each time when the heartbeat interval BI of the driver is estimated, the coarse-graining process about heartbeat intervals and the abnormality sign determination process are executed.

On the other hand, in a case where the duration of the abnormal posture is the second abnormal posture threshold value or greater (YES in step S11), in step S12, the driver abnormal posture determination unit 12 causes the display 31 or the alarm device 32 to output an alarm which notifies an abnormality is occurring to the driver. In addition, in step S13, the driver abnormal posture determination unit 12 controls each unit of the vehicle 1 by the vehicle control device 30 and starts an emergency evacuation for evacuating the vehicle 1 to a shoulder or the like of a road. Subsequently, the driver state estimation process is finished.

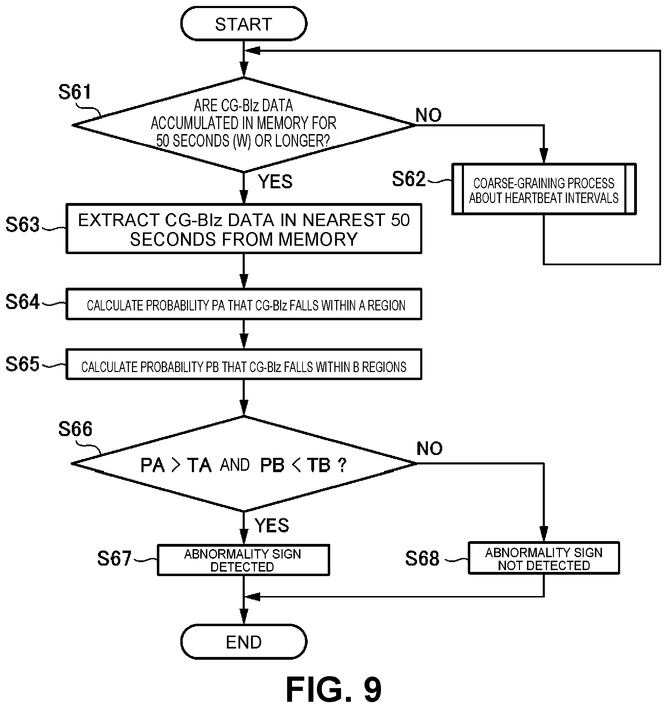

Next, an abnormality sign determination process according to a second embodiment will be described with reference to FIG. 9. FIG. 9 is a flowchart of the abnormality sign determination process according to the second embodiment. Because processes in steps S61 to S64 in the abnormality sign determination process illustrated in FIG. 9 are similar to processes in steps S51 to S54 in the abnormality sign determination process of the embodiment described with reference to FIG. 7, specific descriptions will not be made.

As described with reference to FIG. 8, the abnormality sign of the driver due to failure of the automatic nervous system can be detected based on the difference in the appearance probability distribution of the CG-BIz between the normal group and the abnormal group in the region in the vicinity of the central portion, the region including the average value zero of the CG-BIz. In the second embodiment, focusing on the fact that the appearance probability density of the CG-BIz of the abnormal group is lower than the appearance probability density of the CG-BIz of the normal group in peripheral regions adjacent to the region in the vicinity of the central portion, the region including the average value zero of the CG-BIz, this fact is added to conditions for detection of the abnormality sign of the driver, and precision of abnormality sign detection is thereby made higher.

Specifically, in tail ranges in which absolute values of the CG-BIz are greater than those in the range in the vicinity of the central portion which includes the average value 0 of the CG-BIz (ranges on both sides and outsides of the range in the vicinity of the central portion which includes the average value 0), ranges, in which the 1σ section of the appearance probability distribution of the CG-BIz of the normal group (the band of rightward-upward oblique lines) does not overlap the 1σ section of the appearance probability distribution of the CG-BIz of the abnormal group (the dotted band), (for example, ranges in which the CG-BIz is -0.9 or greater to - 0.35 or smaller and is 0.35 or greater to 0.9 or smaller) are set as B regions (peripheral regions). Furthermore, in a case where the probability PA that the CG-BIz calculated from the heartbeat interval of the driver falls within the A region is higher than the abnormality sign threshold value TA and a probability PB that the CG-BIz calculated from the heartbeat interval of the driver falls within the B regions is lower than an abnormality sign threshold value TB, the driver abnormality sign determination unit 110 determines that the abnormality sign of the driver is detected. The abnormality sign threshold value TB is set to a value which is lower than a probability that the representative value -1σ of the appearance probability distribution of the CG-BIz of the normal group falls within the B regions and which is higher than a probability that the representative value +1σ of the appearance probability distribution of the CG-BIz of the abnormal group falls within the B regions.

That is, the abnormality sign threshold value TB is set to a value which is smaller than a value obtained by integrating the curve representing the representative value -1σ of the appearance probability distribution of the CG-BIz of the normal group (a curve demarcating a lower side of the band of rightward-upward oblique lines) by the B regions (that is, the ranges in which the CG-BIz is -0.9 or greater to -0.35 or smaller and is 0.35 or greater to 0.9 or smaller) and which is greater than a value obtained by integrating the curve representing the representative value +1σ of the appearance probability distribution of the CG-BIz of the abnormal group (a curve demarcating an upper side of the dotted band) by the B regions, and TB = 0.5, for example. Numerical value ranges of the CG-BIz which define the B regions and the abnormality sign threshold value TB are in advance set based on the heart rate data of the normal group and the abnormal group and are stored in the memory 10b.

In step S64 in FIG. 9, the probability PA that the CG-BIz included in the time-series data of the standardized coarse-grained heartbeat interval CG-BIz falls within the A region is calculated, and thereafter in step S65, the driver abnormality sign determination unit 110 calculates the probability PB that the CG-BIz included in the time-series data of the standardized coarse-grained heartbeat interval CG-BIz falls within the B regions.

Next, in step S66, the driver abnormality sign determination unit 110 determines whether or not the probability PA calculated in step S64 is greater than the abnormality sign threshold value TA (that is, the probability TA that the representative value -1σ of the appearance probability distribution of the CG-BIz of the abnormal group falls within the A region) stored in the memory 10b and the probability PB calculated in step S65 is smaller than the abnormality sign threshold value TB (that is, a probability TB that the representative value +1σ of the appearance probability distribution of the CG-BIz of the abnormal group falls within the B regions) stored in the memory 10b.

As a result, in a case where the PA is greater than the TA and the PB is smaller than the TB (YES in step S66), the driver abnormality sign determination unit 110 determines that the abnormality sign of the driver is detected in step S67. On the other hand, in a case where the PA is the TA or smaller or the PB is the TB or greater (NO in step S66), the driver abnormality sign determination unit 110 determines that the abnormality sign of the driver is not detected in step S68. After step S67 or step S68, the process returns to the main routine of the driver state estimation process.

Note that a condition that a time period PB₂ in which the determination that the probability PB is smaller than the abnormality sign threshold value TB continues is longer than an abnormality sign threshold value TB₂ (for example, 25 seconds) which is in advance set and stored in the memory 10b is added to a condition that the PA is greater than the TA and the PB is smaller than the TB in step S66, and the abnormality sign of the driver may thereby be detected.

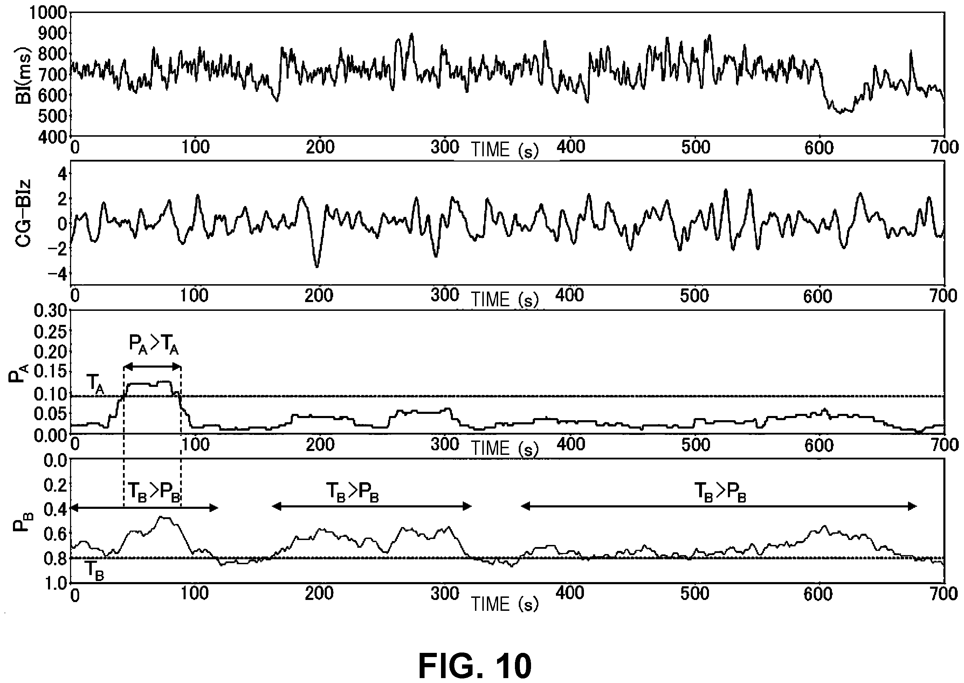

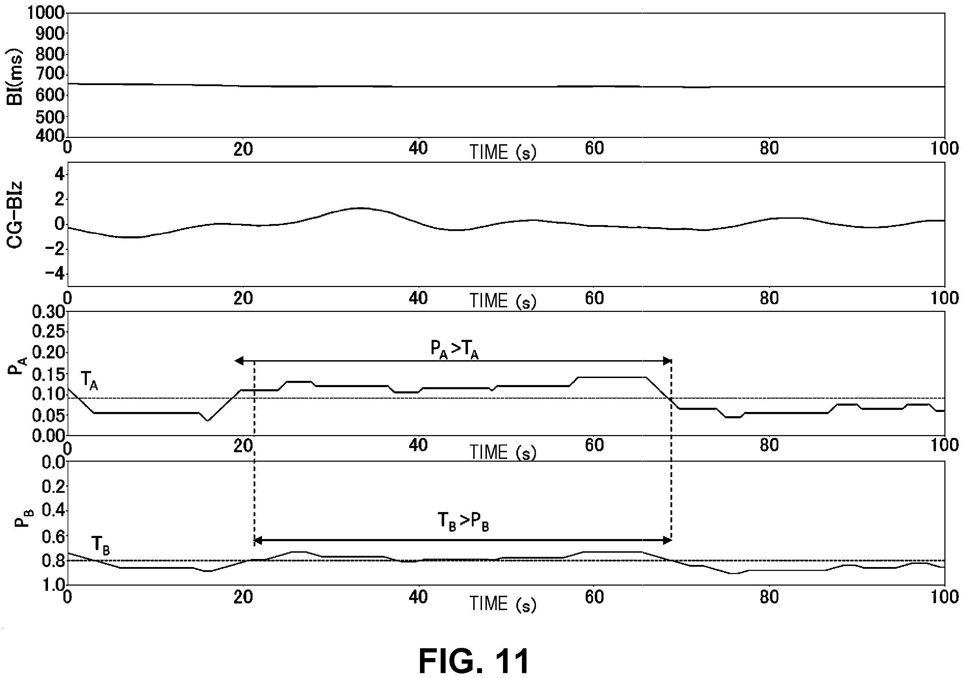

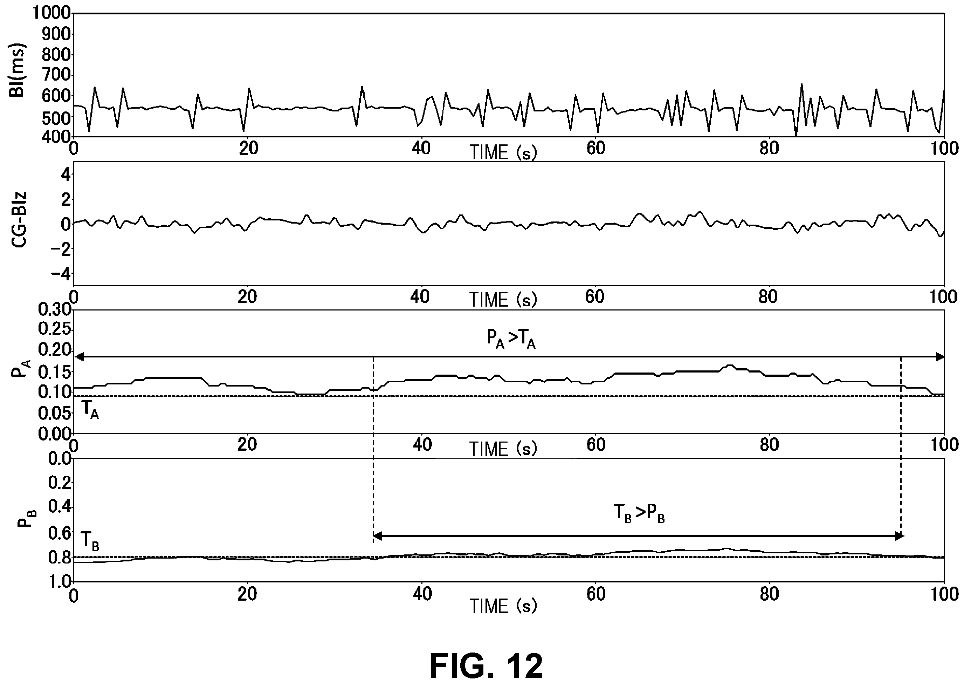

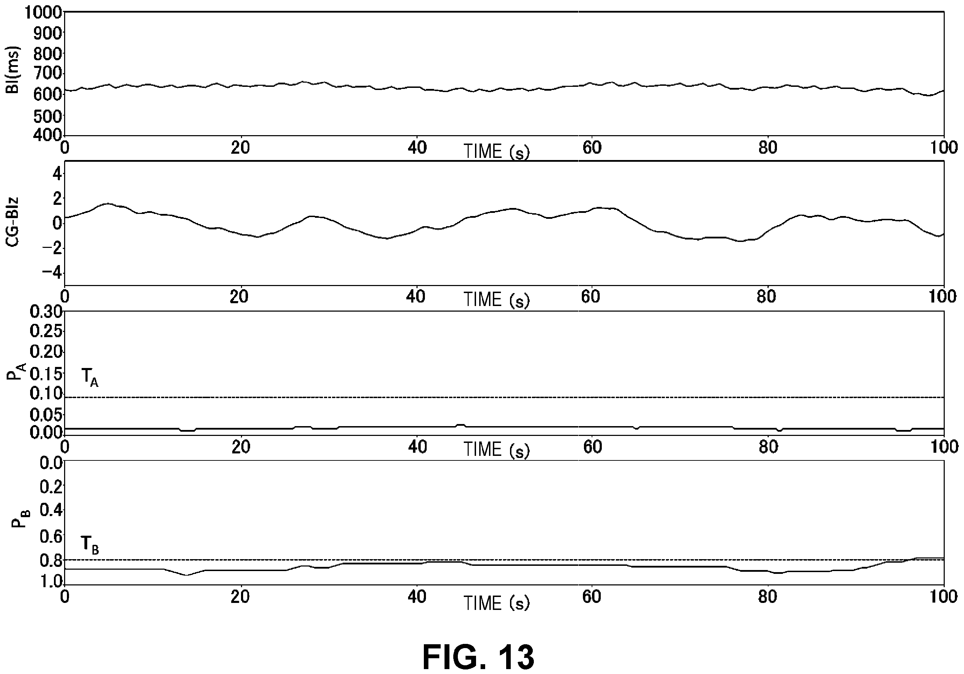

Next, practical examples of the abnormality sign detection for the driver by the abnormality sign detection device 100 of the present embodiment will be described with reference to FIG. 10 and FIG. 13. FIG. 10 to FIG. 13 are time charts illustrating respective time changes in the heartbeat interval BI, the standardized coarse-grained heartbeat interval CG-BIz, and the probability PA and the probability PB that the CG-BIz included in the time-series data of the standardized coarse-grained heartbeat interval CG-BIz falls into the A region and falls into the B regions. In particular, FIG. 10 illustrates a state at a time before an epileptic seizure occurs, FIG. 11 illustrates a state of sympathetic nervous system failure, FIG. 12 illustrates a state of parasympathetic nervous system failure, and FIG. 13 illustrates a state where no abnormality of the automatic nervous system is present.

In an example of epilepsy which is illustrated in FIG. 10, the epileptic seizure occurs at a time point of approximately 600 seconds at which the heartbeat interval BI rapidly lowers, but failure of the parasympathetic nervous system occurs before the seizure. Because a fluctuation in the heartbeat interval BI decreases in response to failure of the parasympathetic nervous system, the probability PA that the CG-BIz included in the time-series data of the standardized coarse-grained heartbeat interval CG-BIz falls into the A region starts to rise from a time point of approximately 20 seconds, and the probability PA exceeds the abnormality sign threshold value TA (0.09 in FIG. 10) at a time point from approximately 30 seconds to approximately 80 seconds. Further, the probability PB that the CG-BIz included in the time-series data of the standardized coarse-grained heartbeat interval CG-BIz falls into the B regions is smaller than the abnormality sign threshold value TB (0.8 in FIG. 10) at a time point from 0 second to approximately 120 seconds, a time point from approximately 160 seconds to approximately 320 seconds, and a time point from approximately 350 seconds to approximately 670 seconds. Consequently, in the example in FIG. 10, the driver abnormality sign determination unit 110 according to the above-described embodiment or the second embodiment detects the abnormality sign of the driver at a time point from approximately 30 seconds to approximately 80 seconds at which the probability PA exceeds the abnormality sign threshold value TA.

Further, in an example of the sympathetic nervous system failure which is illustrated in FIG. 11, an influence on the heartbeat interval BI by the sympathetic nerves disappears, suppression by the parasympathetic nerves becomes dominant, and the fluctuation in the heartbeat interval BI thereby becomes small. As a result, at a time point from approximately 18 seconds to approximately 68 seconds, the probability PA that the CG-BIz included in the time-series data of the standardized coarse-grained heartbeat interval CG-BIz falls into the A region exceeds the abnormality sign threshold value TA (0.09 in FIG. 11). Further, the probability PB that the CG-BIz included in the time-series data of the standardized coarse-grained heartbeat interval CG-BIz falls into the B regions is smaller than the abnormality sign threshold value TB (0.8 in FIG. 11) at a time point from approximately 20 seconds to approximately 68 seconds. Consequently, in the example in FIG. 11, the driver abnormality sign determination unit 110 according to the above-described embodiment detects the abnormality sign of the driver at the time point from approximately 18 seconds to approximately 68 seconds at which the probability PA exceeds the abnormality sign threshold value TA. Further, the driver abnormality sign determination unit 110 according to the second embodiment detects the abnormality sign of the driver at the time point from approximately 20 seconds to approximately 68 seconds at which the probability PA exceeds the abnormality sign threshold value TA and the probability PB becomes smaller than the abnormality sign threshold value TB.

Further, in an example of the parasympathetic nervous system failure (specifically, heart failure) which is illustrated in FIG. 12, an influence on the heartbeat interval BI by the parasympathetic nerves disappears, promotion by the sympathetic nerves becomes dominant, the heartbeat interval BI is separated into relatively large fluctuation and small fluctuation, and an intermediate fluctuation becomes small. As a result, at a time point from 0 second to 100 seconds, the probability PA that the CG-BIz included in the time-series data of the standardized coarse-grained heartbeat interval CG-BIz falls into the A region exceeds the abnormality sign threshold value TA (0.09 in FIG. 12). Further, the probability PB that the CG-BIz included in the time-series data of the standardized coarse-grained heartbeat interval CG-BIz falls into the B regions is smaller than the abnormality sign threshold value TB (0.8 in FIG. 12) at a time point from approximately 35 seconds to approximately 95 seconds. Consequently, in the example in FIG. 12, the driver abnormality sign determination unit 110 according to the above-described embodiment detects the abnormality sign of the driver at the time point from 0 second to approximately 100 seconds at which the probability PA exceeds the abnormality sign threshold value TA. Further, the driver abnormality sign determination unit 110 according to the second embodiment detects the abnormality sign of the driver at the time point from approximately 35 seconds to approximately 95 seconds at which the probability PA exceeds the abnormality sign threshold value TA and the probability PB becomes smaller than the abnormality sign threshold value TB.

Meanwhile, in an example in which no abnormality of the automatic nervous system is present and which is illustrated in FIG. 13, the sympathetic nerves and the parasympathetic nerves work to mutually keep balance, and the heartbeat interval BI thereby fluctuates. As a result, the probability PA that the CG-BIz included in the time-series data of the standardized coarse-grained heartbeat interval CG-BIz falls into the A region does not exceed the abnormality sign threshold value TA (0.09 in FIG. 13), and the probability PB that the CG-BIz falls into the B regions is greater than the abnormality sign threshold value TB (0.8 in FIG. 13). Consequently, in the example in FIG. 13, the driver abnormality sign determination unit 110 according to the above-described embodiment or the second embodiment does not detect the abnormality sign of the driver.

Note that in the above-described embodiments, a description is made about a case where the heartbeat interval estimation unit 101 detects the pulse wave from the video of the body surface of the driver, the video being photographed by the driver camera 20, and estimates the time interval between the adjacent peaks in the pulse wave to be the heartbeat interval BI; however, the pulse wave (heartbeat) may be detected by a device different from the driver camera 20 (such as an electric wave type sensor, an electrostatic capacitance sensor, a smartwatch including a heart rate detection function, or an electrocardiograph, for example), and the heartbeat interval BI may thereby be estimated.

Next, a description will be made about working effects of the above-described driver abnormality sign detection method and device according to the present embodiment.

The abnormality sign detection device 100 estimates the heartbeat interval BI of the driver based on heartbeats detected by the driver camera 20, acquires the time-series data of the heartbeat interval BI of the driver in the predetermined time period 2×S, standardizes the time-series data of the heartbeat interval BI, calculates the probability PA that the standardized coarse-grained heartbeat interval CG-BIz falls within the predetermined range (A region), which has zero as the center, in the time-series data of the standardized coarse-grained heartbeat interval CG-BIz, and determines that the sign of the abnormality of the driver is detected in a case where the probability PA is greater than the predetermined abnormality sign threshold value TA. Thus, the abnormality sign detection device 100 acquires the time-series data of the standardized coarse-grained heartbeat interval CG-BIz even from the time-series data of the heartbeat interval BI in a short time such as about several ten seconds, calculates the probability PA that the CG-BIz falls into the A region, and can thereby detect the abnormality sign. Accordingly, the abnormality sign of the driver can be detected while both of high responsiveness and high reliability are realized.

Further, the predetermined range (A region) which has zero as the center is included in the central region where the peak of the appearance probability distribution of the standardized coarse-grained heartbeat interval CG-BIz acquired from the subject group of subjects having abnormalities in the automatic nervous systems protrudes higher than the peak of the appearance probability distribution of the standardized coarse-grained heartbeat interval CG-BIz acquired from the subject group of subjects with normal automatic nervous systems. Thus, a determination about the abnormality sign can be made in a region where clear differences are present between a case where an abnormality is present in the automatic nervous system and a normal case. Accordingly, reliability of the abnormality sign detection for the driver can be enhanced.

Further, the abnormality sign threshold value TA is greater than a value obtained by integrating, by the predetermined range (A region) which has zero as the center, the appearance probability distribution of the standardized coarse-grained heartbeat interval CG-BIz acquired from the subject group of subjects with normal automatic nervous systems, and is smaller than a value obtained by integrating, by the predetermined range (A region) which has zero as the center, the appearance probability distribution of the standardized coarse-grained heartbeat interval CG-BIz acquired from the subject group of subjects having abnormalities in the automatic nervous systems. Thus, a boundary between the case where an abnormality is present in the automatic nervous system and the normal case in the predetermined range (A region) which has zero as the center is set as the abnormality sign threshold value TA, and a determination about the abnormality sign can thereby be made. Accordingly, reliability of the abnormality sign detection for the driver can be enhanced.

Further, because the coarse-graining process about heartbeat intervals and the abnormality sign determination process are executed at each time when the heartbeat interval BI of the driver is estimated, in a case where the sign of an abnormality of the automatic nervous system of the driver occurs, the sign of the abnormality can quickly be detected. Accordingly, the abnormality sign of the driver can be detected while both of high responsiveness and high reliability are realized.

Further, because heartbeats of the driver are detected by the driver camera 20 which photographs the body surface of the driver, without using a special device for heartbeat detection, the abnormality sign detection for the driver can be performed at a low cost and without a load to the driver.

### Reference Signs List:

- 1: vehicle
- 10: driver state estimation device
- 10a: processor
- 10b: memory
- 11: head posture estimation unit
- 12: driver abnormal posture determination unit
- 13: abnormal posture threshold value
- 20: driver camera
- 30: vehicle control device
- 31: display
- 32: alarm device
- 40: vehicle actuator
- 41: lamps
- 42: horn
- 100: abnormality sign detection device
- 101: heartbeat interval estimation unit
- 102: driver individual authentication unit
- 103: individual parameter selection unit
- 104: driver-specific first parameter
- 105: driver-specific second parameter
- 106: first parameter learning unit
- 107: second parameter learning unit
- 108: heartbeat interval coarse-graining process unit
- 109: buffer
- 110: driver abnormality sign determination unit
- 111: abnormality sign threshold value

## Claims

1. A driver abnormality sign detection method for detecting a sign of an abnormality of a driver who drives a vehicle (1), the driver abnormality sign detection method comprising:
a step A of detecting heartbeats of the driver by a heartbeat detection device;
a step B of estimating a heartbeat interval of the driver by a processor (10a) based on the heartbeats detected by the heartbeat detection device;
a step C of acquiring time-series data of the heartbeat interval of the driver in a predetermined time period by the processor;
a step D of standardizing the time-series data of the heartbeat interval by the processor (10a);
a step E of calculating, by the processor (10a), a probability that the standardized heartbeat interval falls within a predetermined range, which has zero as a center, in the time-series data of the standardized heartbeat interval; and
a step F of determining, by the processor (10a), that the sign of the abnormality of the driver is detected in a case where the probability is greater than a predetermined abnormality sign threshold value (111).

2. The driver abnormality sign detection method according to claim 1, wherein
the predetermined range, which has zero as the center, is included in a central region where a peak of appearance probability distribution of a standardized heartbeat interval acquired from a subject group of subjects having abnormalities in automatic nervous systems protrudes higher than a peak of appearance probability distribution of a standardized heartbeat interval acquired from a subject group of subjects with normal automatic nervous systems.

3. The driver abnormality sign detection method according to claim 2, wherein
the abnormality sign threshold value (111) is greater than a value obtained by integrating, by the predetermined range which has zero as the center, the appearance probability distribution of the standardized heartbeat interval acquired from the subject group of subjects with normal automatic nervous systems, and is smaller than a value obtained by integrating, by the predetermined range which has zero as the center, the appearance probability distribution of the standardized heartbeat interval acquired from the subject group of subjects having abnormalities in the automatic nervous systems.

4. The driver abnormality sign detection method according to any one of claims 1 to 3, wherein
the step C, the step D, the step E, and the step F are executed at each time when the heartbeat interval is estimated in the step B.

5. A driver abnormality sign detection device detecting a sign of an abnormality of a driver who drives a vehicle, the driver abnormality sign detection device comprising:
a heartbeat detection device which detects heartbeats of the driver;
a memory (10b) which stores a program; and
a processor (10a) which executes the program, wherein
the processor (10a) is configured to
estimate a heartbeat interval of the driver based on the heartbeats detected by the heartbeat detection device,
acquire time-series data of the heartbeat interval of the driver in a predetermined time period,
standardize the time-series data of the heartbeat interval,
calculate a probability that the standardized heartbeat interval falls within a predetermined range, which has zero as a center, in the time-series data of the standardized heartbeat interval, and
determine that the sign of the abnormality of the driver is detected in a case where the probability is greater than a predetermined abnormality sign threshold value (111).

6. The driver abnormality sign detection device according to claim 5, wherein
the heartbeat detection device is a driver camera (20) which photographs a body surface of the driver.

7. The driver abnormality sign detection device according to claim 5 or 6 wherein
a driver state estimation device (10) comprises a driver camera (20), a vehicle control device (30), a display (31) and preferably an alarm device (32).

8. The driver abnormality sign detection device according to claim 6, further comprising:
a vehicle actuator (40) which is able to actuate a driving power source, a brake, and a steering device of the vehicle (1) in accordance with the control by the vehicle control device (30).

9. The driver abnormality sign detection device according to one of the claims 5 to 8, wherein
the processor (10a) functions as a head posture estimation unit (11), a driver abnormal posture determination unit (12), and an abnormality sign detection device (100).

10. The driver abnormality sign detection device according to claim 9, wherein
the abnormality sign detection device (100) includes a heartbeat interval estimation unit (101) and a driver individual authentication unit (102).

11. The driver abnormality sign detection device according to claim 10, wherein
the abnormality sign detection device (100) further includes an individual parameter selection unit (103), a first parameter learning unit (106), and a second parameter learning unit (107

12. The driver abnormality sign detection device according to claim 11, wherein
the abnormality sign detection device (100) further includes a heartbeat interval coarse-graining process unit (108), a buffer (109), and a driver abnormality sign determination unit (110).

13. The driver abnormality sign detection device according to one of the claims 5 to 12, further comprising
a memory (10b) of the driver state estimation device (10) storing an abnormal posture threshold value (13), at least one driver specific parameter (104, 105), and an abnormality sign threshold value (111).
